# EUROPÄISCHE PATENTSCHRIFT

(11) **EP 1 111 050 B1**
(45) Veröffentlichungstag und Bekanntmachung des Hinweises auf die Patenterteilung: **14.03.2007**
(21) Anmeldenummer: 00125719.5
(22) Anmeldetag: 24.11.2000
(51) Int. Cl.: C12N 15/16, C07K 14/585, C12N 5/10, A61K 38/23, G01N 33/74, C07K 16/26

(54) **Lösungen von humanem Procalcitonin**
Solutions of human procalcitonin
Solutions de procalcitonine humaine

(30) Priorität: 22.12.1999 DE 19962434; 03.04.2000 DE 10016278; 08.06.2000 DE 10027954
(43) Veröffentlichungstag der Anmeldung: 27.06.2001
(73) Patentinhaber: Dade Behring Marburg GmbH, 35001 Marburg (DE)
(72) Erfinder: Althaus, Harald, 35083 Wetter (DE); Hauser, Hans Peter, Dr., 35041 Marburg (DE)

(56) Entgegenhaltungen:
- EP-A- 0 997 735
- EP-A- 1 026 506
- US-A- 3 057 782
- US-A- 5 993 811
- NYLEN E S ET AL: "MORTALITY IS INCREASED BY PROCALCITONIN AND DECREASED BY AN ANTISERUM REACTIVE TO PROCALCITONIN IN EXPERIMENTAL SEPSIS" CRITICAL CARE MEDICINE, Bd. 26, Nr. 6, Juni 1998 (1998-06), Seiten 1001-1006, XP000879293 ISSN: 0090-3493
- REHLI MICHAEL ET AL: "Molecular cloning and expression of mouse procalcitonin." BIOCHEMICAL AND BIOPHYSICAL RESEARCH COMMUNICATIONS, Bd. 226, Nr. 2, 1996, Seiten 420-425, XP002167870 ISSN: 0006-291X
- LE MOULLEC, J. M. ET AL: "The complete sequence of human preprocalcitonin" FEBS LETT. (1984), 167(1), 93-7 , XP000993494
- LIU, XIAO-QING ET AL: "A novel method for increasing production of mature proteins in the periplasm of Escherichia coli" PROTEIN SCI. 8(10), 2085-2089 , Oktober 1999 (1999-10), XP000993517
- KARZAI W ET AL: "PROCALCITONIN - A NEW INDICATOR OF THE SYSTEMIC RESPONSE TO SEVERE INFECTIONS" INFECTION, Bd. 25, Nr. 6, November 1997 (1997-11), Seiten 329-334, XP000879253 ISSN: 0300-8126
- WRENGER ET AL: "Amino-terminal truncation of procalcitonin, a marker for systemic bacterial infections, by dipeptidyl peptidase IV (DP IV)" FEBS LETTERS, Bd. 466, 21. Januar 2000 (2000-01-21), Seiten 155-159, XP002143500 ISSN: 0014-5793

### Sequenzprotokoll

<110> Dade Behring Marburg GmbH
<120> Humanes Procalcitonin, dessen Herstellung und Verwendung
<130> Ma1236
<140>
   <141>
<150> 19962434.8
   <151> 1999-12-22
<150> 10016278.9
   <151> 2000-04-03
<150> 10027954.6
   <151> 2000-06-08
<160> 15
<170> PatentIn Ver. 2.1
<210> 1
   <211> 101
   <212> DNA
   <213> Unbekannt
<220>
   <223> Beschreibung des unbekannten Organismus: Primer, nicht genomische DNA
<400> 1
   gtgggatccg caccattcag gtctgccctg gagagcagcc cagcagaccc ggccacgctc 60
   agtgaggacg aagcgcgcct cctgctggct gcactggtgc a 101
<210> 2
   <211> 104
   <212> DNA
   <213> Unbekannt
<220>
   <223> Beschreibung des unbekannten Organismus: Primer, nicht genomische DNA
<400> 2
   gtgaagctta gatctggggc tgtccaggct ggagccctct ctctcttgct cctgctccag 60
   ctcactggcc ttcatctgca catagtcctg caccagtgca gcca 104
<210> 3
   <211> 18
   <212> DNA
   <213> Unbekannt
<220>
   <223> Beschreibung des unbekannten Organismus: Primer, nicht genomische DNA
<400> 3
   gtgggatccg caccattc 18
<210> 4
   <211> 20
   <212> DNA
   <213> Unbekannt
<220>
   <223> Beschreibung des unbekannten Organismus: Primer, nicht genomische DNA
<400> 4
   gtgaagctta gatctggggc 20
<210> 5
   <211> 18
   <212> DNA
   <213> Unbekannt
<220>
   <223> Beschreibung des unbekannten Organismus: Primer, nicht genomische DNA
<400> 5
   gtgtctagat ctaagcgg 18
<210> 6
   <211> 18
   <212> DNA
   <213> Unbekannt
<220>
   <223> Beschreibung des unbekannten Organismus: Primer, nicht genomische DNA
<400> 6
   gtgaagcttt tagttggc 18
<210> 7
   <211> 116
   <212> PRT
   <213> Unbekannt
<220>
   <223> Beschreibung des unbekannten Organismus: Protein, hum. Procalcitonin
<400> 7
   Ala Pro Phe Arg Ser Ala Leu Glu Ser Ser Pro Ala Asp Pro Ala Thr
    1               5                  10                  15
   Leu Ser Glu Asp Glu Ala Arg Leu Leu Leu Ala Ala Leu Val Gln Asp
               20                  25                  30
   Tyr Val Gln Met Lys Ala Ser Glu Leu Glu Gln Glu Gln Glu Arg Glu
           35                  40                  45
   Gly Ser Ser Leu Asp Ser Pro Arg Ser Lys Arg Cys Gly Asn Leu Ser
       50                  55                  60
   Thr Cys Met Leu Gly Thr Tyr Thr Gln Asp Phe Asn Lys Phe His Thr
   65                  70                  75                  80
   Phe Pro Gln Thr Ala Ile Gly Val Gly Ala Pro Gly Lys Lys Arg Asp
                   85                  90                  95
   Met Ser Ser Asp Leu Glu Arg Asp His Arg Pro His Val Ser Met Pro
               100                 105                 110
   Gln Asn Ala Asn
           115
<210> 8
   <211> 116
   <212> PRT
   <213> Unbekannt
<220>
   <223> Beschreibung des unbekannten Organismus: Protein, hum. Procalcitonin
<400> 8
   Ala Pro Phe Arg Ser Ala Leu Glu Ser Ser Pro Ala Asp Pro Ala Thr
    1               5                  10                  15
   Leu Ser Glu Asp Glu Ala Arg Leu Arg Leu Ala Ala Leu Val Gln Asp
                20                  25                  30
   Tyr Val Gln Met Lys Ala Ser Glu Leu Glu Gln Glu Gln Glu Arg Glu
            35                  40                  45
   Gly Ser Ser Leu Asp Ser Pro Arg Ser Lys Arg Cys Gly Asn Leu Ser
        50                  55                  60
   Thr Cys Met Leu Gly Thr Tyr Thr Gln Asp Phe Asn Lys Phe His Thr
    65                  70                  75                  80
   Phe Pro Gln Thr Ala Ile Gly Val Gly Ala Pro Gly Lys Lys Arg Asp
                    85                  90                  95
   Met Ser Ser Asp Leu Glu Arg Asp His Arg Pro His Val Ser Met Pro
               100                 105                 110
   Gln Asn Ala Asn
           115
<210> 9
   <211> 128
   <212> PRT
   <213> Unbekannt
<220>
   <223> Beschreibung des unbekannten Organismus: Protein, hum. Procalcitonin
<400> 9
   Met Arg Gly Ser His His His His His His Gly Ser Ala Pro Phe Arg
    1               5                  10                  15
   Ser Ala Leu Glu Ser Ser Pro Ala Asp Pro Ala Thr Leu Ser Glu Asp
               20                  25                  30
   Glu Ala Arg Leu Arg Leu Ala Ala Leu Val Gln Asp Tyr Val Gln Met
           35                  40                  45
   Lys Ala Ser Glu Leu Glu Gln Glu Gln Glu Arg Glu Gly Ser Ser Leu
       50                  55                  60
   Asp Ser Pro Arg Ser Lys Arg Cys Gly Asn Leu Ser Thr Cys Met Leu
   65                  70                  75                  80
   Gly Thr Tyr Thr Gln Asp Phe Asn Lys Phe His Thr Phe Pro Gln Thr
                   85                  90                  95
   Ala Ile Gly Val Gly Ala Pro Gly Lys Lys Arg Asp Met Ser Ser Asp
               100                 105                 110
   Leu Glu Arg Asp His Arg Pro His Val Ser Met Pro Gln Asn Ala Asn
           115                 120                 125
<210> 10
   <211> 116
   <212> PRT
   <213> Unbekannt
<220>
   <223> Beschreibung des unbekannten Organismus: Protein, hum. Procalcitonin
<400> 10
   Ala Pro Phe Arg Ser Ala Leu Glu Ser Ser Pro Ala Asp Pro Ala Thr
    1               5                  10                  15
   Leu Ser Glu Asp Glu Ala Arg Leu Leu Leu Ala Ala Leu Val Gln Asp
               20                  25                  30
   Tyr Val Gln Met Lys Ala Ser Glu Leu Glu Gln Glu Gln Glu Arg Glu
           35                  40                  45
   Gly Ser Ser Leu Asp Ser Pro Arg Ser Lys Arg Cys Gly Asn Leu Ser
       50                  55                  60
   Thr Cys Met Leu Gly Thr Tyr Thr Gln Asp Phe Asn Lys Phe His Thr
   65                  70                  75                  80
   Phe Pro Gln Thr Ala Ile Gly Val Gly Ala Pro Gly Lys Lys Arg Asp
                   85                  90                  95
   Met Ser Ser Asp Leu Glu Arg Asp His Arg Pro His Val Ser Met Pro
               100                 105                 110
   Gln Asn Ala Asn
           115
<210> 11
   <211> 116
   <212> PRT
   <213> Unbekannt
<220>
   <223> Beschreibung des unbekannten Organismus: Protein, hum. Procalcitonin
<400> 11
   Ala Pro Phe Arg Ser Ala Leu Glu Ser Ser Pro Ala Asp Pro Ala Thr
    1               5                  10                  15
   Leu Ser Glu Asp Glu Ala Arg Leu Arg Leu Ala Ala Leu Val Gln Asp
               20                  25                  30
   Tyr Val Gln Met Lys Ala Ser Glu Leu Glu Gln Glu Gln Glu Arg Glu
           35                  40                  45
   Gly Ser Ser Leu Asp Ser Pro Arg Ser Lys Arg Cys Gly Asn Leu Ser
       50                  55                  60
   Thr Cys Met Leu Gly Thr Tyr Thr Gln Asp Phe Asn Lys Phe His Thr
   65                  70                  75                  80
   Phe Pro Gln Thr Ala Ile Gly Val Gly Ala Pro Gly Lys Lys Arg Asp
                   85                  90                  95
   Met Ser Ser Asp Leu Glu Arg Asp His Arg Pro His Val Ser Met Pro
               100                 105                 110
   Gln Asn Ala Asn
           115
<210> 12
   <211> 128
   <212> PRT
   <213> Unbekannt
<220>
   <223> Beschreibung des unbekannten Organismus: Protein, hum. Procalcitonin
<400> 12
   Met Arg Gly Ser His His His His His His Gly Ser Ala Pro Phe Arg
    1               5                  10                  15
   Ser Ala Leu Glu Ser Ser Pro Ala Asp Pro Ala Thr Leu Ser Glu Asp
               20                  25                  30
   Glu Ala Arg Leu Arg Leu Ala Ala Leu Val Gln Asp Tyr Val Gln Met
           35                  40                  45
   Lys Ala Ser Glu Leu Glu Gln Glu Gln Glu Arg Glu Gly Ser Ser Leu
       50                  55                  60
   Asp Ser Pro Arg Ser Lys Arg Cys Gly Asn Leu Ser Thr Cys Met Leu
   65                  70                  75                  80
   Gly Thr Tyr Thr Gln Asp Phe Asn Lys Phe His Thr Phe Pro Gln Thr
                   85                  90                  95
   Ala Ile Gly Val Gly Ala Pro Gly Lys Lys Arg Asp Met Ser Ser Asp
               100                 105                 110
   Leu Glu Arg Asp His Arg Pro His Val Ser Met Pro Gln Asn Ala Asn
           115                 120                 125
<210> 13
   <211> 3462
   <212> DNA
   <213> Unbekannt
<220>
   <223> Beschreibung des unbekannten Organismus: Vektorsequenz, DNA
i <400> 13
<210> 14
   <211> 363
   <212> DNA
   <213> Unbekannt
<220>
   <223> Beschreibung des unbekannten Organismus:DNA Sequenz, hum. Procalcitonin
<400> 14
   ggatccgcac cattcaggtc tgccctggag agcagcccag cagacccggc cacgctcagt 60
   gaggacgaag cgcgcctccg gctggctgca ctggtgcagg actatgtgca gatgaaggcc 120
   agtgagctgg agcaggagca agagagagag ggctccagcc tggacagccc cagatctaag 180
   cggtgcggta atctgagtac ttgcatgctg ggcacataca cgcaggactt caacaagttt 240
   cacacgttcc cccaaactgc aattggggtt ggagcacctg gaaagaaaag ggatatgtcc 300
   agcgacttgg agagagacca tcgccctcat gttagcatgc cccagaatgc caactaaaag 360
   ctt                                                               363
<210> 15
   <211> 351
   <212> DNA
   <213> Unbekannt
<220>
   <223> Beschreibung des unbekannten Organismus:DNA Sequenz, hum. Procalcitonin
<400> 15
   gcaccattca ggtctgccct ggagagcagc ccagcagacc cggccacgct cagtgaggac 60
   gaagcgcgcc tcctgctggc tgcactggtg caggactatg tgcagatgaa ggccagtgag 120
   ctggagcagg agcaagagag agagggctcc agcctggaca gccccagatc taagcggtgc 180
   ggtaatctga gtacttgcat gctgggcaca tacacgcagg acttcaacaa gtttcacacg 240
   ttcccccaaa ctgcaattgg ggttggagca cctggaaaga aaagggatat gtccagcgac 300
   ttggagagag accatcgccc tcatgttagc atgccccaga atgccaacta a          351

## Beschreibung

Die Erfindung betrifft stabilisierte Lösungen enthaltend humanes Procalcitonin, sowie mittels solcher Lösungen hergestellte Kontrollen und Standards.

Procalcitonin ("pCT") ist ein aus 116 Aminosäuren bestehendes Protein mit einem Molekulargewicht von ca. 13000 Dalton. Es stellt das Prohormon von Calcitonin dar, welches unter normalen Stoffwechselbedingungen in den C-Zellen der Schilddrüse gebildet und sezerniert wird. Die Synthese von pCT und Calcitonin beginnt mit der Translation eines 141 Aminosäuren umfassenden Vorläuferpeptides, dem Preprocalcitonin ("Pre-pCT"). Die Aminosäuresequenz von menschlichem Pre-pCT wurde 1984 von Moullec et al. in FEBS Letters, 167:93-97 beschrieben. Nach Abspaltung des Signalpeptides (ersten 25 Aminosäuren von Pre-pCT) entsteht pCT. Bei Gesunden wird durch spezifische Proteolyse aus pCT intrazellulär das Hormon Calcitonin (Aminosäuren 60-91 der pCT-Aminosäuresequenz) sowie das N-Procalcitonin (Aminosäuren 1-57 der pCT-Aminosäuresequenz) und das Katacalcin (Aminosäuren 96-116 der pCT-Aminosäuresequenz) gebildet (s.a. Conlon et al. (1988) Biochem. J., 256:245-250). Das pCT sowie dessen Fragmente wurden insbesondere bei bestimmten Tumorerkrankungen (Ghillani et al. (1989) Cancer Research, 49:6845-6851) sowie bei Sepsis (EP-B1-0 656 121) und SIRS ("systemic inflammatory response syndrome") (Snider et al. (1997) J. Investig. Med., 45:552-560) in erhöhter Konzentration im Serum bzw. Plasma von Patienten nachgewiesen.

Bei der typischen Sepsis werden Bakterien von einem Herd aus ständig oder schubweise in die Blutbahn abgegeben. Die klinischen Erscheinungen werden durch Endotoxin oder durch andere pyrogene und toxische Substanzen in ihrer Interaktion mit Körpermechanismen verursacht. Der akute Einbruch löst Schüttelfrost und in schweren Fällen eine Schockreaktion aus. Sonderformen des septischen Schocks sind das Waterhouse-Friderichsen-Syndrom und das Toxinschocksyndrom (TSS). Das TSS ist als ein akutes Krankheitsbild bei Staphylokokken-Infektionen bekannt, das durch ein spezielles Staphylokokken-Toxin hervorgerufen wird. Eine schwere Sepsis entwickelt sich relativ häufig bei Patienten mit schweren Grunderkrankungen wie z.B. Tumorerkrankungen, schweren Verbrennungen und Traumen,

Die Bedeutung des Nachweises der Erreger im Blut ("positive Blutkultur, Bakteriämie") ist für die Sepsisdiagnose in den Hintergrund getreten, da in der Regel nur in 20 bis 40% der Sepsisfälle die Blutkultur positiv ist. Der Sepsisbegriff hat sich deshalb einem Wandel unterzogen. Der moderne Begriff Sepsis" beschreibt ein klinisches Syndrom, welches in der Regel Fieber, Leukozytose, Bewußtseinsveränderungen, einen hyperdynamischen Kreislauf ("warmer Schock") und einen hypermetabolischen Zustand umfaßt, wobei eine positive Blutkultur als Voraussetzung für die Diagnose einer Sepsis nicht mehr erforderlich ist.

In WO 98/33524 wird vorgeschlagen, Antikörper, die an pCT binden, zur Therapie von Sepsis und SIRS einzusetzen.

Über lange Jahre wurden polyklonale Antikörper, die durch Immunisierung mit Calcitonin gewonnen wurden, zum Nachweis des sogenannten immunreaktiven Calcitonins, welches neben Calcitonin auch das Procalcitonin und weitere Bruchstücke des Procalcitonins umfaßt, verwendet. Durch die Immunisierung mit synthetischen Peptiden, deren Aminosäuresequenzen mit den Sequenzen von Teilabschnitten des Procalcitonins übereinstimmt, gelang es verschiedene monoklonale Antikörper herzustellen, die an verschiedene Epitope des Calcitonins und Katacalcins binden (Ghillani et al. (1988) J. Immunol., 141:3156-3163).

Auf Basis dieser Antikörper wurden auch Sandwich-Immunoassays zum Nachweis von pCT bzw. Calcitonin in Serumproben entwickelt. Zum Nachweis von Calcitonin-Vorläufermolekülen wurde eine Kombination eines Anti-Katacalcin-Antikörpers mit einem Anti-Calcitonin-Antikörper vorgeschlagen. Als Standardmaterial wurde ein auf diese Antikörper abgestimmtes synthetisches Peptid eingesetzt.

Es ist bekannt, daß das Meßsignal von Standards und Proben bei immunchemischen Testen nicht zwangsläufig auch bei absolut gleicher Antigenmenge identisch sein muß. Sind Standard- und Probenantigen nicht wirklich hinsichtlich ihrer immunchemischen Reaktivität identisch, werden die im Test eingesetzten Antikörper entweder das eine oder das andere Antigen besser erkennen. Dies führt letzlich zu unterschiedlichen Meßsignalen von Proben und Standards.

Hieraus folgt, daß die Verwendung von Antigenbruchstücken als Standardantigen anstatt des gesamten Proteins häufig mit Nachteilen verbunden ist, insbesondere kann dies zu verfälschten Meßwerten führen. Ferner können die auf der räumlichen Struktur des richtig gefalteten Proteins beruhenden Epitope in der Regel mit kürzeren Peptiden nicht richtig dargestellt werden. Dies hat zur Folge, daß bei der Verwendung von Peptiden als Immunogen keine Antikörper gegen solche Konformationsepitope gewonnen werden können. Auch gerade in kompetitiven Testformaten ist es vorteilhaft, wenn die nachzuweisende Substanz die gleiche immunchemische Reaktivität wie das entsprechende Festphasen- oder Label-gebundene Testreagenz aufweist.

Obwohl die komplette Aminosäuresequenz von humanem pCT bereits seit 1984 bekannt war, ist es zunächst nicht gelungen, humanes pCT insbesondere in größeren Mengen und reproduzierbar herzustellen. Zunächst konnte einzig murines pCT mittels gentechnischer Verfahren in E. coli exprimiert werden (Rehli et al. (1996) Biochem Biophys. Res. Com., 226: 420-425).

Das murine pCT unterscheidet sich von humanem pCT jedoch so deutlich (etwa 77% Homologie auf Aminosäureebene), daß sich für den Fachmann weiterhin die Aufgabe stellt, ein Verfahren zu entwickeln, welches erlaubt humanes pCT in größeren Mengen, kostengünstig und in isolierter Form herzustellen, um dieses insbesondere als Immunogen und/oder als Standard- und Kontrollserenantigen einsetzen zu können.

In der vor dem internationalen Anmeldetag der vorliegenden Anmeldung aber nach dem ältesten Prioritätstag der vorliegenden Anmeldung veröffentlichten Europäischen Patentanmeldung EP-1 026 506 A1 wurde schließlich die rekombinante Herstellung auch eines humanen Procalcitonins offenbart sowie dessen Verwendung als Standard nach Zusatz bestimmter Substanzen zur Stabilisierung.

Außerdem wurde in der vor dem internationalen Anmeldetag der vorliegenden Anmeldung aber nach dem ältesten Prioritätstag der vorliegenden Anmeldung veröffentlichten Europäischen Patentanmeldung EP-0 997 735 A2 über die Stabilisierung bestimmter biologischer Flüssigkeiten wie beispielsweise Serum, Plasma, Liquor, Pleuraexudate oder Ascitesflüssigkeit berichtet.

Außerdem wude in dem US-Patent Nr. 3 057 782 ein quervemetztes Gelatineprodukt offenbart sowie seine Verwendung als Blutplasmaersatz.

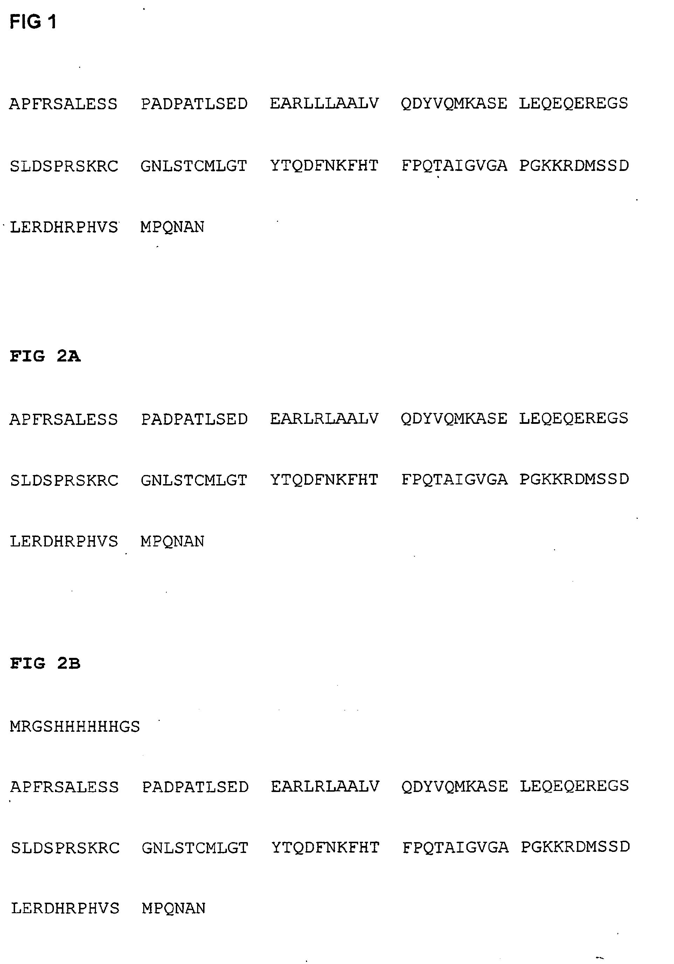

Procalcitonin (pCT) entsprechend vorliegender Offenbarung ist als isoliertes Polypeptid herstellbar, insbesondere mit Hilfe gentechnischer Verfahren, welches die Aminosäuresequenz von humanem pCT enthält. Die Aminosäuresequenz von humanem pCT ist in FIG 1 dargestellt.

Unter dem Begriff "gentechnische Verfahren" sind im Sinne dieser Offenbarung insbesondere auch Verfahren zu verstehen, bei denen das zu exprimierende Polypeptid von eukaryontischen oder prokaryontischen Zellen produziert wird, wobei die für das zu exprimierende Polypeptid kodierende Nukleinsäuresequenz, rekombinante Nukleinsäuresequenzen miteingeschlossen, in diese Zellen z.B. mittels Vektoren, Liposomen, Projektilen oder Co-Präzipation mit Salzen vorher eingebracht wurde. Bei einem anderen Verfahren wird ein bereits in der Zelle natürlicherweise vorhandenes Gen, das das zu exprimierende Polypeptid kodiert, durch aktivierende Maßnahmen, z.B. durch Genamplifikation oder durch Aktivierung mittels artifiziell eingebrachter Promotor- und/oder Enhancer-Sequenzen oder durch Deletion von Repressor-bindenden Sequenzen, so aktiviert, daß die Zelle das zu exprimierende Polypeptid in größerer Menge als natürlicherweise exprimiert.

Unter dem Begriff "Aminosäuresequenz von humanem pCT" sind im Sinne dieser Offenbarung auch durch Austausch, Deletion oder Hinzufügen von Aminosäuren leicht abgewandelte Aminosäuresequenzen zu verstehen, wobei diese Änderungen keinen gravierenden negativen Einfluß auf die Bindungseigenschaften des Polypeptids gegenüber Anti-pCT-Antikörpern haben sollen. Der Fachmann kann dies anhand von entsprechenden Bindungsstudien mit vorhandenen Anti-pCT-Antikörpern überprüfen.

Der Begriff "Peptide" im Sinne dieser Offenbarung umfaßt Säureamide, die bei Hydrolyse in Aminosäuren zerfallen, beispielsweise Aminosäurepolymere wie z.B. Polypeptide, Oligopeptide, Proteine oder Proteinfragmente. Sind nicht mehr als zehn Aminosäuren miteinander verknüpft, so spricht man in der Regel von Oligopeptiden, darüber hinaus von Polypeptiden.

Weitere offenbarungsgemäße Polypeptide sind isolierte Polypeptide, die die in FIG 2A oder 2B dargestellte Aminosäuresequenz enthalten und insbesondere mit Hilfe gentechnischer Verfahren hergestellt wurden.

Ein offenbarungsgemäßes Verfahren ist auch ein Verfahren zur Herstellung von humanem Procalcitonin, wobei (i) ein Gen kodierend für ein Polypeptid, das die Aminosäuresequenz von humanem Procalcitonin, enthält, in einen Vektor eingefügt wird, (ii) ein Wirtsorganismus mit diesem Gen-enthaltenden Vektor transformiert wird und (iii) das von dem Wirtsorganismus exprimierte Polypeptid isoliert wird. Besondere Varianten diese Verfahrens sind solche, bei denen ein Polypeptid mit der Aminosäuresequenz gemäß FIG 1, 2A oder 2B hergestellt wird

Ein "Vektor" ist insbesondere ein DNA- oder RNA-Molekül, das in einem Wirtsorganismus zur Replikation in der Lage ist und aus dem man durch Einbau eines oder mehrerer fremder Gene ein rekombiniertes DNA- oder RNA-Molekül konstruieren kann. Beispiele für gebräuchliche Vektoren sind bakterielle Plasmide; virale Genome, insbesondere die Genome von Bakteriophagen; Hefechromosomen u. -plasmide, insbesondere YEp, Ylp, YRp, YAC; Ti-Plasmid; sowie von Adenoviren, Papillomaviren und Retroviren abgeleitete Vektoren. Zur Ermöglichung der Expression des fremden Gens verfügt ein Vektor in der Regel über einen Promotor, welcher möglichst physikalisch oder chemisch induzierbar die Transkription einer Boten-RNA initiiert, welche für das zu exprimierende Protein kodiert. Ferner weist ein Vektor in der Regel eine Nukleinsäuresequenz auf, die einen Transkriptionsstop bewirkt, und Nukleinsäuresequenzen, die eine möglichst effiziente Translation bewirken, wie beispielsweise bei bakterieller Expression eine Ribosomen-Bindungsstelle. Außerdem sollte ein Expressionsvektor über Translationsstop-Sequenzen in allen möglichen Leserastern verfügen.

Andere offenbarungsgemäße Verfahren sind dadurch gekennzeichnet, daß der Vektor, z.B. pQE-30, einen Fusionsanteil, bevorzugt Poly-Histidin, kodiert, welcher später eine einfache Aufreinigung des Procalcitonin-Fusionsproteins erlaubt.

Geeignete Wirtszellen für die offenbarungsgemäßen Verfahren sind menschliche, tierische, pflanzliche oder prokaryontische Zellen, darunterinsbesondere E. coli. Zellen.

Unter einem "Fusionsprotein" ist im Sinne dieser Offenbarung ein Protein zu verstehen, das sich aus pCT und entweder C- oder N-terminat einem weiteren Poly- oder Oligopeptid zusammensetzt, welches insgesamt als ein Polypeptid translatiert wird. Der Fusionsanteil sollte bevorzugt entweder die Exprimierbarkeit des Procalcitonins erhöhen und/oder eine spätere einfache affinitätschromatographische Aufreinigung des Fusionsproteins ermöglichen.

Insbesondere wird in den offenbarungsgemäßen Verfahren zur Isolierung des offenbarungsgemäßen Polypeptids Metallaffinitätschromatographie und/oder Gelfiltration eingesetzt werden.

Bei der Metallaffinitätschromatographie wird die Tatsache ausgenutzt, daß eine Chromatographie-Gelmatrix, welche Chelat gebundene zweiwertige Metallionen, beispielsweise Ni²⁺ , enthält und wobei noch mehrere Koordinationsstellen des Metallions frei zugänglich sind, mit Proteinen, welche mehrere Histidine in Folge enthalten, eine reversible Bindung eingehen kann. Eine schonende Elution des Poly-Histidinpolypeptids kann dann kompetitiv beispielsweise durch imidazolhaltige Puffer erreicht werden.

Ein weiterer Gegenstand vorliegender Offenbarungist ein Plasmid, welches eine oder mehrere Nukleinsäuresequenzen enthält, die eines oder mehrere der offenbarungsgemäßen Polypeptide kodieren. Ein ganz besonders geeignetes Plasmid mit der internen Bezeichnung pQE-PCT wurde am 16.12.1999 bei der DSMZ Deutsche Sammlung von Mikroorganismen und Zellkulturen GmbH, Mascheroder Weg 1b, Braunschweig, Deutschland, mit der Hinterlegungsnummer DSM 13203 hinterlegt.

Gegenstand vorliegender Erfindung sind stabile pCT-Lösungen, die beispielsweise als Kontrollen, Standards oder auch für andere in vitro sowie in vivo Anwendungen genutzt werden können. "Stabil" bedeutet in diesem Zusammenhang, daß die gewünschte Eigenschaft des in der Lösung enthaltenen Procalcitonins, z.B. die Bindungsfähigkeit an bestimmte Antikörper, über einen bestimmten Zeitraum insbesondere bei Flüssiglagerung weitgehend unverändert bleibt, während sich diese Eigenschaft bei "instabilen" pCT-Lösungen im gleichen Zeitraum deutlich verändert.

Stabile pCT-Lösungen lassen sich herstellen, indem (I) ein offenbarungsgemäßes Polypeptid, (II) Sterinester und (III) Polygeline einer serum/plasmahaltigen oder serum/plasmafreien Matrix zugefügt werden.

Insbesondere bei als pCT-Kontrollen und/oder als pCT-Standards eingesetzten pCT-Lösungen kann das eingesetzte pCT auch z.B. ein aus natürlichen Quellen isoliertes oder rekombinant hergestelltes Peptid sein, welches zumindest wesentliche Teilbereiche der Aminosäuresequenz von humanen pCT enthält, wie zum Beispiel größere Spaltprodukte von pCT. Das Peptid muß aber in einem Verfahren zum quantitativen oder qualitativen Nachweis von pCT als Standard- und/oder Kontrollserumantigen geeignet sein.

Die zur Herstellung der erfindungsgemäßen pCT-Lösung geeigneten Sterinester gehören zu der Stoffklasse der Steroide (Gonanderivate) die allgemein eine Hydroxygruppe in der 3 β Position kennzeichnet. Wesentliche Unterschiede bestehen in der in 17(20) Stellung haftenden Seitenkette. Sterine stellen eine große Klasse dar (BEYER et al. (1981), Lehrbuch der organischen Chemie, S. 649-664 "Steroide"). Vorteilhafterweise können Vitamin D3, Oestron, Stigmasterin und besonders vorteilhaft Cholesterin und dessen Derivate verwendet werden.

Die erfindungsgemäß einzusetzenden Sterinester besitzen bevorzugt außerdem eine über eine Dicarbonsäure gekoppelte Polyethylenglykolgruppe (PEG Gruppe). Prinzipiell können alle bekannten Dicarbonsäuren verwendet werden; besonders vorteilhaft ist die Verwendung von Bernsteinsäure, Adipinsäure oder Sebacinsäure.

Die PEG Gruppe soll im wesentlichen auch die Löslichkeit des Sterinesters gewährleisten, so daß der Fachmann, gegebenenfalls durch ein Experiment, die optimale Länge leicht bestimmen kann. Erfahrungsgemäß sind folgende Kettenlängen vorteilhaft: Polyethylenglycol 600, Polyethylenglycol 900 oder Polyethylenglycol 3000.

Zur Herstellung der erfindungsgemäßen pCT-Lösungen werden bevorzugt Sterinester der allgemeinen Formel I eingesetzt:

R₁-O -(O)C-R₂-C(O)-O-[CH₂-CH₂-O]ₙ-CH₂-CH₂ -OH Formel I

worin n = 1-200 und
R₁ = Sterin ist
R₂ = aliphatischer oder aromatischer Ring mit 4 bis 8 C-Atomen, von denen mindestens eines durch N, S oder O ersetzt werden kann, oder eine geradlinige oder verzweigte Kette mit 0 bis 12 C-Atomen darstellt, besonders bevorzugt ist die Verwendung der Sterinester in denen R₁ eine Verbindung der allgemeinen Formel II ist: 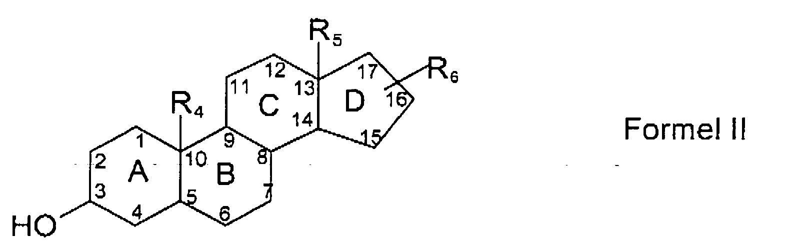 worin R₄ und R₅ H oder -CH₃ sein können,
R₆ eine geradkettige oder verzweigte Kette mit 1 bis 12 C-Atomen, eine -OH oder =O Gruppe sein kann,
die Ringe A,B, C und D jeder für sich gesättigt, ungesättigt, oder aromatisch sein können,
und, wenn R₄ = -C(19)H₃ ist, der Ring B zwischen C(9) und C(10) unter Ausbildung einer Doppelbindung zwischen C(9) und C(19) geöffnet sein kann.

Ganz besonders bevorzugt ist die Verwendung von Sterinestern, wobei der Sterinrest von Cholesterin, Vitamin D3, Stigmasterin oder Oestron stammt.

Vorteilhafterweise wird der Sterinester in einer solchen Konzentration zugesetzt, daß die Konzentration in der pCT-Lösung 0.05 - 5 Gew. %, bevorzugterweise 0.1-3 Gew. %, besonders bevorzugterweise 0.5- 1.5 Gew. % beträgt.

Die erfindungsgemäßen pCT-Lösungen können auch Proteinaseinhibitoren, z.B. Aprotinin, Benzamidin, Bestatin, Cystatin, Pepstatin, PMSF, Trypsin Inhibitor, und/oder Detergenzien, insbesondere nicht-ionische und/oder zwitter-ionische Detergenzien, enthalten.

Polygeline ist eine Mischung aus thermisch abgebauten und vernetzten Gelatineproteinen und kann hergestellt werden nach der deutschen Auslageschrift 1155134 oder dem US Patent 3057782. Vorteilhafterweise wird Polygeline in einer solchen Konzentration zugesetzt, daß die Konzentration in der pCT-Lösung 0.1 - 10 Gew% bevorzugterweise 1 - 8 Gew% besonders bevorzugterweise 2 - 6 Gew% beträgt.

Zur Messung des Stabilisierungseffektes - insbesondere bei als Standards und/oder Kontrollen dienenden pCT-Lösungen - kann beispielsweise pCT in einem nephelometrischen Meßverfahren bestimmt werden.

Eine weitere Ausführungsform dieser Erfindung beinhaltet die Verwendung der erfindungsgemäßen Standards, Kontrollen und pCT-Lösungen in Verfahren zum quantitativen oder qualitativen Nachweis von pCT, Calcitonin, Katacalcin, N-Procalcitonin und/oder anderen pCT-Fragmenten in einer Probe.
FIG 1 zeigt die Aminosäuresequenz von humanen pCT
FIG 2 zeigt die Aminosäuresequenz eines offenbarungsgemäßen Polypeptids ohne (A) und mit (B) Fusionsanteil.

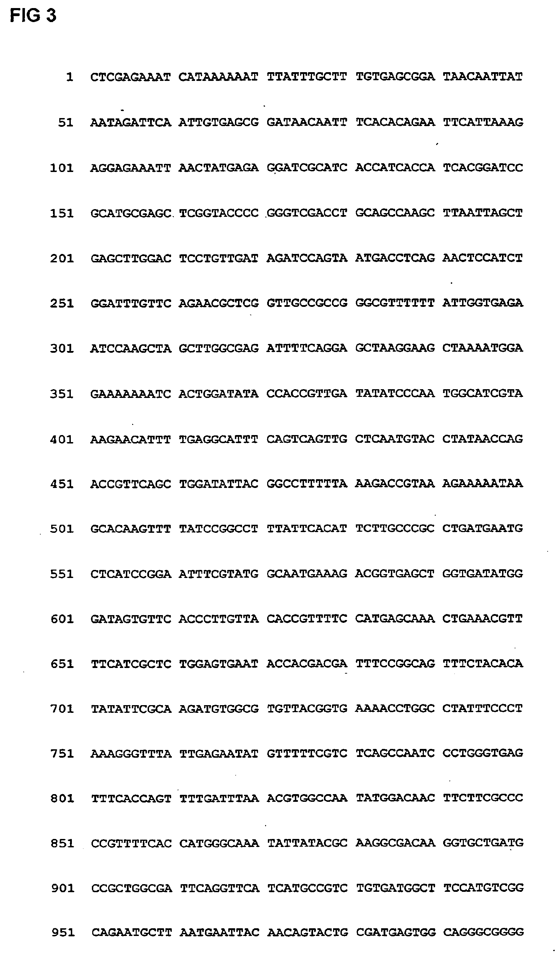

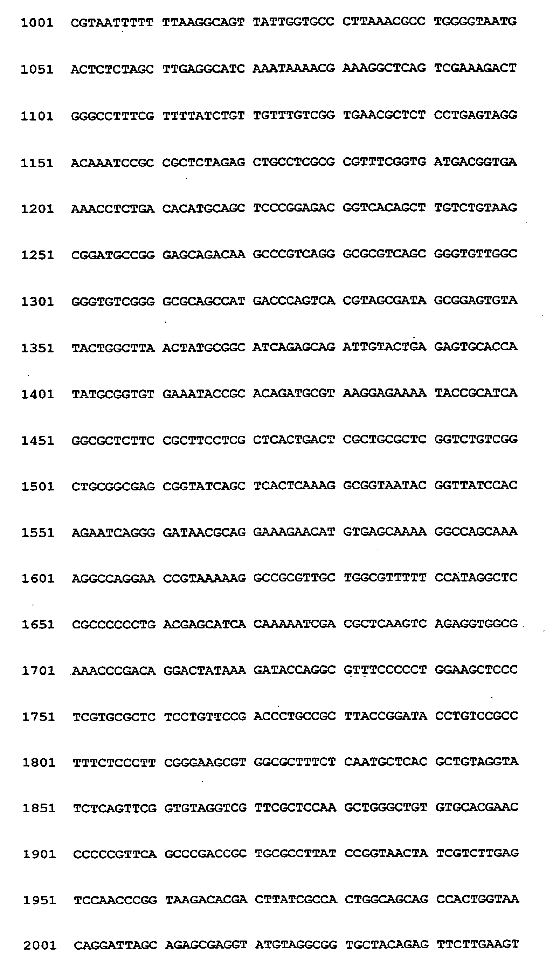

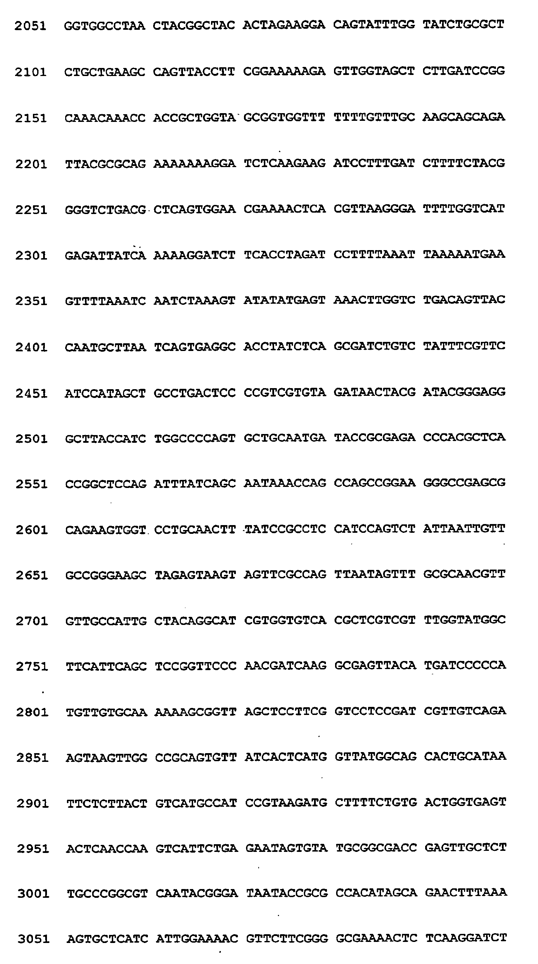

FIG 3 zeigt die Nukleinsäuresequenz des Vektors pQE-30 (3462 Basenpaare).
FIG 4 zeigt die Nukleinsäuresequenz des pCT kodierenden Inserts welches in pQE-30 kloniert wurde einschließlich der verwendeten Schnittstellen.
Fig 5 zeigt die Nukleinsäuresequenz von humanem pCT

Die im folgenden beschriebenen Beispiele dienen zur exemplarischen Beleuchtung einzelner Aspekte dieser Erfindung und sind nicht als Einschränkung zu verstehen.

### BEISPIELE:

### 1. Klonlerung von Procalcitonin

Der N-Terminus von pCT (siehe FIG 1) wurde mittels synthetischer Oligonukleotide konstruiert, während der C-Terminus mittels PCR (Polymerase Chain Reaction) auf Basis genomischer humaner plazentärer DNA durchgeführt wurde:

### (i) N-Terminus

Verwendet wurden als Primer die beiden folgenden Oligonukleotide:

Wobei die jeweils 16 3'-terminalen Nukleotide zueinander komplementär waren.

Es wurde folgende PCR durchgeführt:

In einem 50 µl -Reaktionsansatz wurden 0,25 mM dNTP (Amersham Pharmacia, Freiburg, Deutschland) je 1 µM der Primer 1094 und 1095, 10 mM Tris HCl, pH 8.3, 50mM KCI, 1.5 mM MgCl₂, 0,001 % Gelatine, 2,5 U Ampli-Taq-DNA-Polymerase (Perkin Elmer, Branchburg, New Jersey, USA) pipettiert und nach folgendem Temperaturprogramm mittels des Thermocyclers "GenAmp 9700" von Perkin Elmer (auch alle weiteren PCR-Reaktionen wurden auf demselben Gerät durchgeführt) amplifiziert:
Initial: 5' 94°C
5x der Zyklus: 94°C 30sec, 52°C 30sec und 72°C 30sec
Terminal: 72°C 10min.

5 µl dieses Ansatzes wurden in einem neuen 50 µl Ansatz wie oben jedoch unter Verwendung der Primer 1098 und 1099
1098: 5' GTG GGA TCC GCA CCA TTC 3'
1099:5' GTG AAG CTT AGA TCT GGG GC 3'
und nach folgendem Temperaturprogramm durchgeführt:
Initial: 5' 94°C
30x der Zyklus: 94°C 30sec, 56°C 30sec und 72°C 30sec
Terminal; 72°C 10min.

5 µl dieses Ansatzes wurden mit 0,5µg des Vektors pCR2.1 des TA-Cloning Kits von Invitrogen nach Angaben des Herstellers ligiert und in E.coli INVαF' transformiert. Das neukonstruierte Plasmid wurde nach Standardmethoden isoliert und sequenziert, dabei wurde ein Aminosäureaustausch von L zu R an Position 37 des Procalcitonins gefunden.

### (ii) C-Terminus

Nach Standardmethoden (alle als Standardmethoden beschriebenen Protokolle stammen aus "Current Protocols in Molecular Biology", 1995, Wiley & Sons, Inc. New York, USA) wurde aus 2g humanem Plazentagewebe genomische DNA isoliert und als Template für die folgende PCR eingesetzt:

In einem 50 µl -Reaktionsansatz wurden laut Herstellerangaben zu dem Taq PCR Core Kit Cat.No.:201223 (Qiagen, Hilden, Deutschland) als Template 0,5 µg (1µl) gen. hum. DNA , und als Primer je 1 µg der Primer 1100 und 1101 hinzu pipettiert
1100: 5' GTG TCT AGA TCT AAG CGG 3'
1101: 5' GTG AAG CTT TTA GTT GGC 3'
und nach folgendem Temperaturprogramm amplifiziert:
Initial: 3' 94°C
30x der Zyklus: 94°C 30sec, 45°C 30sec und 72°C 30sec
Terminal: 72°C 10min.

5 µl dieses Ansatzes wurden mit 0,5 µg des Vektors pCR2.1 des TA Cloning Kits von Invitrogen nach Angaben des Herstellers ligiert und in E.coli INVαF' transformiert. Das neukonstruierte Plasmid wurde nach Standardmethoden isoliert und sequenziert, hierbei konnte das Vorliegen der Wildtypsequenz bestätigt werden.

### (iii) Konstruktion des Expressionsplasmids

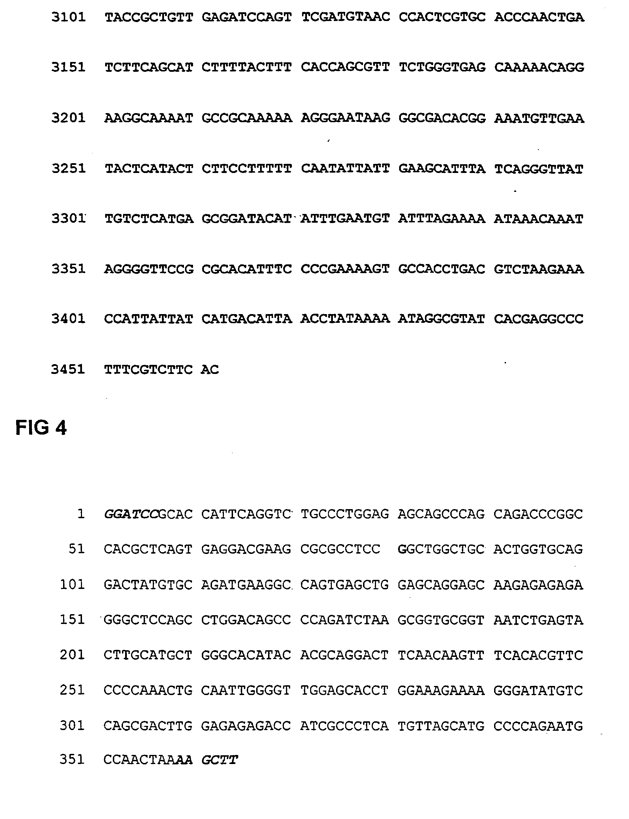

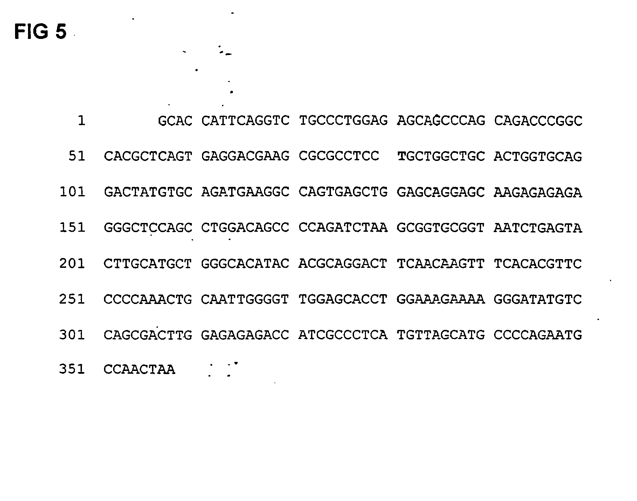

1 µg des Vektors pQE-30 (Qiagen) wurde mit den Restriktionsendonukleasen BamHl und HindIII (sämtliche Restriktionsendonukleasen wurden von Boehringer Mannheim, Mannheim, Deutschland, bezogen) geöffnet. Weiter wurde aus dem Plasmid pCR2.1 welches den N-Terminus enthielt, der N-Terminus mittels BamHl und HindIII (BamHl und HindIII waren artifiziell über die PCR eingeführt worden) ausgeschnitten, nach Standardmethoden isoliert und in den geöffneten Vektor pQE-30 ligiert. Das Konstukt wurde isoliert und mittels BgIII und HindIII geöffnet (BgIII kommt natürlicherweise am 3'Ende des N-Terminus vor und konnte hier zur Kontruktion eingesetzt werden). Schließlich wurde aus dem Vektor pCR2.1 welcher den C-Terminus enthielt, der C-Terminus mittels BgIII und HindIII ausgeschnitten, nach Standardmethoden isoliert und in den mit BgIII und HindIII geöffneten Vektor pQE-30 welcher schon den N-Terminus enthielt, ligiert. Der das korrekte Plasmid enthaltende Klon wurde identifiziert und das Konstrukt durch Sequenzierung verifiziert. Das von BamHl bis HindIII reichende für die Konstruktion verwendete Insert ist in Fig. 4, die natürliche humane pCT Sequenz in Fig. 5 gezeigt.

### 2. Expression von Procalcitonin

Die Expression von Procalcitonin wurde zunächst im Kleinstmaßstab von 1ml durchgeführt:

1 ml LB-Medium (Current Protocols in Molecular Biology) welches 50 µg Ampicillin (Sigma, Deisenhofen, Deutschland) enthielt, wurde mit einer Einzelkolonie des Expressionsplasmids im E.coli Stamm JM109 angeimpft und bei einer OD₆₀₀ von 0,4 mit 1 mM IPTG (Isopropyl-Thio-galaktosid, Sigma) für 2h induziert.

Unerwarteterweise wurde dabei eine starke Expression des Fusionsproteins von humanem pCT mit dem N-Teminus des pQE Vektors MRGSHHHHHHGS bei Analyse des Gesamtproteins der Kultur in einem Coomassie gefärbten PAGE-Gel (Current Protocols in Molecular Biology) gefunden.

Hierauf wurde die Expression in einem größerem Maßstab nach Standardbedingungen durchgeführt, d.h. von einem Einzelklon wurde eine Übernachtkultur in 100 ml LB-Medium, welche 50µg/ml Ampicillin enhielt angelegt und bei 37°C geschüttelt. Diese stationär gewachsene Kultur wurde 1:50 in 1l frisches LB-Medium (Ampicillin 50 µg/ml) verdünnt, weiter bei 37°C geschüttelt und bei einer OD₆₀₀ von 0,6 mit 1 mM IPTG für 3 h induziert.

Überraschenderweise wurde dabei ein drastischer Einbruch bzw. eine völlige Einstellung der Expression des Fusionsproteins festgestellt. Dieses negative Ergebnis ließ sich reproduzieren, was darauf schließen läßt, daß das Fusionsprotein für E.coli toxisch wirkt und sehr schnell eine Selektion auf Mutanten erfolgt, die das Fusionsprotein nicht oder nur sehr schlecht exprimieren. Es wurde daher versucht, die Expression zu optimieren.

Variiert wurde dabei der Expressionsstamm (JM109, M15, BL21 und W3110 (Stratagene, LaJolla, USA)), die Stärke des Selektionsdruck über die Variation der Ampicillin Konzentration, die Stärke der Induktion über Variation des Induktors IPTG und die Expressionsdauer durch Verfolgung des zeitlichen Verlaufs der Induktionsstärke nach der Induktion.

Gefunden wurden dabei die folgenden optimalen Parameter:

Frisch mit dem Expressionsplasmid transformierte JM109 werden über Nacht bei 37°C unter Schütteln in LB-Medium mit 100µg/ml Ampicillin angezogen und dann 1:50 in 1l frischem LB-Medium (Ampicillin 100µg/ml) verdünnt und weiter bei 37°C geschüttelt, und bei einer OD₆₀₀ von 0,4 mit 2mM IPTG für 3h induziert.

Bei Einhaltung dieser optimierten Bedingungen wurden reproduzierbar ca. 13mg Fusionsprotein aus 1l Kultur nach einer Reinigung unter nativen Bedingungen über Metallaffinitätschromatographie nach Angaben des Herstellers (Talon Metal Affinity Resin, Clontech, Palo Alto, USA) und anschließender Gelfiltration über Superdex^{™} 75 HiLoad (Amersham Pharmacia), erhalten.

### 3.) Aminoterminale Sequenzierung

Die aminoterminale Sequenz des rekombinaten humanen Procalcitonins wurde durch automatischen Edmanabbau an einem Applied Biosystems 477A Sequenzer bestimmt.

Die gefundene aminoterminale Sequenz ist nach der Sequenz des pQE Vektors Identisch zu der gefundenen von pCT aus einem humanen Schilddrüsentumor (J.M. Conlon et al. (1988) Biochm. J. 256: 245-250 (s. Tabelle 1)).

**Tabelle1: Aminoterminale Sequenz von humanen pCT und rek. humanen pCT**

| Protein | Aminosäurensequenz | Referenz |
|---|---|---|
| Rek. hum. pCT | M-R-G-S-H-H-H-H-H-H-G-S-A-P-F-R-S-A-L-E-S-S-P | diese Erfindung |
| | * | |

| | | |
|---|---|---|
| * pQE Vektor-Fusionsanteil (unterstrichen) | | |

### 4. Massenspektrometrische Bestimmung der relativen Molekülmasse

Die Bestimmung der relativen Molekülmasse des hergestellten rekombinanten Procalcitonins erfolgte mittels Elektrospray-Massenspektrometrie. Das nach Expression und Aufreinigung erhaltene rekombinante pCT wurde gegen dest. Wasser dialysiert und in einer Konzentration von 50 µg/ml in Methanol / Wasser / Essigsäure (50/50/0.1) vermessen. (Orifice-Spannung 90 V; lonenspray-Voltage 5000 V).

Die aus dem erhaltenen Spektrum berechnete mittlere Molekülmasse von rekombinanten humanen pCT beträgt 14235± 2 Dalton.

Das Ergebnis ist in sehr guter Übereinstimmung mit der auf Basis der theoretischen Aminosäurensequenz (J.M. Conlon et al. (1988) Biochem. J., 256: 245-250) und unter Berücksichtigung des pQE Vektor-Fusionanteils (pQE Vektor: MRGSHHHHHHGS) und des Aminosäurenaustauschs an Position 37 (L zu R) berechneten theoretischen Masse von 14239 Dalton und bestätigt somit die Expression von rekombinanten humanen pCT.

### 5. Bestimmung der Reaktivität im LUMltest^{®} PCT

Der LUMltest^{®} PCT (B.R.A.H.M.S, Berlin) ist ein immunoluminometrischer Assay zur Bestimmung von Procalcitonin. Dabei werden zwei monoklonale Antikörper die das Procalcitonin an zwei verschiedenen Stellen (dem Calcitonin- und dem Katacalcin-Anteil) binden, eingesetzt.

Nach Durchführung des Tests gemäß Testbeschreibung des Herstellers werden die Lumineszenzsignale in einem dafür geeigneten Luminometer ermittelt. Die Größe des Lumineszenzsignals ist der PCT-Konzentration der jeweiligen Probe direkt proportional. Über die Lumineszenzsignal-Werte der mitgeführten Standards läßt sich eine Standardkurve erstellen, an der die unbekannte Procalcitonin-Konzentration der Probe abgelesen werden kann.

Das nach Expression und Aufreinigung erhaltene rekombinante pCT wurde in zwei verschiedenen Verdünnungen im LUMltest^{®} PCT, nach Arbeitsanleitung des Herstellers, bestimmmt (s. Tabelle 2).

**Tabelle 2: An einem BeriLux^{®} Analyzer 250 bestimmte Meßwerte (RLU = Relative Light Units)**

| **Probe** | **RLU** | **ng pCT/ml** |
|---|---|---|
| Standard S1 | 72 | 0,08 |
| Standard S2 | 169 | 0,49 |
| Standard S3 | 467 | 1,94 |
| Standard S4 | 6643 | 20,5 |
| Standard S5 | 97085 | 212 |
| Standard S6 | 215415 | 527 |
| | | |
| Rek. PCT Verd. 1 | 18742 | 50,41 |
| Rek. PCT Verd. 2 | 1056 | 4,46 |

Unter Berücksichtigung der beiden eingesetzten Verdünnungen (1:10 000 und 1:100 000) errechnet sich für die beiden untersuchten Proben folgender pCT-Gehalt nach LUMltest^{®} PCT.
Rek. PCT Verd 1: 0,5041 mg/ml
Rek. PCT Verd 2: 0,4463 mg/ml

Diese Untersuchung untermauert die Identität des rekombinanten pCT mit humanen pCT und zeigt die Verwendbarkeit von rek. pCT zum Beispiel als Standard- und/oder Kontrollserenmaterial in einem diagnostischen Assay zur Bestimmung von humanen pCT.

### 6. Herstellung monoklonaler Antikörper gegen rekombinantes pCT

### (i) Immunisierung von Mäusen

BALB/c Mäuse werden mit 20 µg rek. pCT in kompletten Freund'schen Adjuvants intraperitoneal immunisiert. Nach 4 Wochen erfolgt ein Booster mit 20µg rek. pCT in inkompletten Freund'schen Adjuvants (Fa. ICN Biomedical GmbH, Eschwege, Deutschland) und nach 8 Wochen mit 20µg rek. pCT ohne Freund'schen Adjuvants. Die letzten 3 Tage vor der Fusion werden die Mäuse intravenös mit je 20 µg rek. pCT geboostert.

### (II) Fusion

Nach dem Töten der Mäuse durch CO₂-Inhalation wurden die Milzen entnommen und Einzelzellsuspensionen in serumfreien Dulbeccos modifiziertem Eagle Medium (DMEM, Fa. CC Pro GmbH, Neustadt/W, Deutschland) hergestellt. Die Zellen wurden zentrifugiert (g-Zahl 652) und 2 x in DMEM gewaschen. Anschließend wurde die Zellzahl mittels Trypanblau-Färbung bestimmt. Zu etwa 10⁸ Milzzellen wurden 2x10⁷ Myelomzellen (Sp2/0) gegeben. Nach dem Zentrifugieren (g-Zahl 360) wurde der Überstand verworfen, 1ml Polyethylenglycol-Lösung (PEG 4000, Fa. Merck Eurolab, Bruchsal, Deutschland; ca. 50%ig in DMEM) auf das Zellpellet gegeben und nach Resuspension 1 Minute bei 37°C inkubiert. Anschließend wurde tropfenweise ca. 10 ml DMEM zugegeben und 2 bis 4 Minuten bei Raumtemperatur inkubiert. Die fusionierten Zellen wurden abzentrifugiert (g-Zahl 326) und das Pellet in DMEM + 20% FKS (fötales Kälberserum, Fa. BioWhittaker Europe, Verviers, Belgien) + HAT-Lösung (Fa. CC Pro GmbH, Neustadt/W, Deutschland) resuspendiert und in 24 Well-Zellkulturplatten (Fa. Costar) abgefüllt. Die ungefähre Zellkonzentration pro Well betrug 5x10⁴ bis 5x10⁶ Zellen.

2 - 3 Wochen später wurden die entstandenen Zellkolonien (Hybride) entnommen und in neue Kulturplatten überführt.

### (iii) Bestimmung der Spezifität

Die Spezifität der in die Zellkultur abgegebenen Antikörper wurden in einem ersten Testschritt mit Hilfe von Immunisierungsantigen -beschichteten Mikrotiterplatten (Fa. Nunc, Typ B), Beschichtung 0,2 µg/ml ≈ 0,003 µg/Vertiefung, getestet.

In jede Vertiefung der Mikrotitterplatte wurden 100 µl Zellkulturüberstand (Verdünnung 1:2) pipettiert und 1 Stunde bei +15 bis +25°C inkubiert. Nach zweimaligen Waschen der Platte mit Waschlösung-POD (OSEW; Fa. Dade Behring, Marburg, Deutschland) wurden in jede Vertiefung 100µl anti-Maus lgG/F(ab')₂-POD-Konjugat (Fa. Dade Behring, Marburg, Deutschland) eingefüllt und 1 Stunde bei +15 bis +25°C inkubiert. Nach weiterem zweimaligen Waschen der Platte wurde in jede Vertiefung 100µl Chromogen TMB-Lösung (Fa. Date Behring, Marburg, Deutschland) eingefüllt und weitere 30 Minuten bei +15 bis +25°C inkubiert. Nach der Inkubation wurde in jede Vertiefung 100µl Stopplösung POD (Fa. Dade Behring, Marburg, Deutschland) eingefüllt und die Mikrotitterplatte am BEP II (Behring-ELlSA-Prozessor II) bei 450 nm ausgewertet.

In einem 2. Testschritt wurden die Hybride wie oben beschrieben überprüft mit Hilfe von Mikrotiterplatten (Fa. Nunc, Typ B), die mit folgenden Peptiden beschichtet waren:
i. Rekombinantes humanes pCT (0,03 µg/Vertiefung)
ii. Calcitonin human RSA-Konjugat (0,5 µg/Vertiefung, Fa, Bachem, Prod. Nr.: H-2250)
iii. Katacalcin human (PDN-21) RSA-Konjugat (0,5µg/Vertiefung, Fa, Peninsula, Prod. Nr.: 6004)
iv. Calcitonin N-Terminal Flanking Peptide RSA-Konjugat (0,5 µg/Vertiefung, Fa, Bachem, Prod. Nr.: H-3076) = humanes N-Procalcitonin

Die Ergebnisse sind in Tabelle 3 aufgelistet.

**Tabelle 3: Bestimmung der Antikörperspezifität durch Auswertung der Mikrotiterplatten am BEP II (Behring-ELISA-Prozessor II) bei 450 nm.**

| | Extinktion bei 450 nm | | | |
|---|---|---|---|---|
| Hybrid/(Klon) | Rekombinantes humanes Procalcitonin | Calcitonin | Katacalcin | N-Procalcitonin |
| 99-41/14(032) | 2,5 | 0,834 | 0,068 | 0,025 |
| 99-41/5 (05) | 2,5 | 0,010 | 2,5 | 0,014 |
| 99-246/58 | 2,5 | 0,032 | 0.030 | 2,246 |

### (iv) Klonierung

Einzelne Zellen von Hybriden, die die monoklonalen Antikörper produzieren (Bindung an humanes pCT), wurden mit einem Mikromanipulator (Fa. Leitz, Wetzlar, Deutschland) kloniert

### (v) Bestimmung der Antikörpersubklasse

Die Subklasse des Antikörpers wird mittels IsoStrip™-Mouse Monoclonal Antibody Isotyping Kit- der Fa. Boehringer Mannheim, Deutschland, bestimmt.

### (vi) Produktion der Antikörper

Für die Produktion größerer Mengen der monoklonalen Antikörper werden die entsprechenden Zellklone in Rollerflaschen (Fa. Corning Costar Deutschland, Bodenheim) überführt, und bis zum gewünschten Endvolumen bei +37°C expandiert. Danach wird die Rollerkultur-Suspension zur Entfernung der Zellen über 0,22 µm filtriert. Die jetzt zellfreie Antikörperlösung wird über Ultrafilter (Trenngrenze 30 Kilodalton) ankonzentriert und anschließend aufgereinigt.

### (vii) Reinigung der Antikörper

Die erhaltene Antikörperlösung wird gegen 0,14 M Phosphatpuffer pH 8,6 umgepuffert und auf ein mit rProtein A Sepharose Fast Flow (Fa. Amersham Pharmacia) gefüllte Chromatographiesäule aufgetragen (pro 10 mg zu reinigender Antikörper werden 1ml rProtein A Sepharose Fast Flow eingesetzt). Alle nicht gebundenen Komponenten werden durch Waschen der Säule mit 0,14 M Phosphatpuffer pH 8,6 entfernt. Der gebundene Antikörper wird mit 0,1 M Zitronensäure pH 3,0 von der Säule eluiert und gegen 0,05 M Natriumacetat + 0,5 M NaCl + 0,05 M Tris + 0,01% Natriumazid pH 7,0 dialysiert.

### 7. Nachweis von pCT in einer Probe

### (i) Bindung von MAK an Latexpartikel

An Latexpartikel hergestellt nach EP-0246 446 mit einem Durchmesser von 250 bis 310 nm wurde jeweils ein erfindungsgemäßer monoklonaler Antikörper gegen Calcitonin und ein erfindungsgemäßer monoklonaler Antikörper gegen Katacalcin gebunden:

Das verwendete Latexpolymerisat wurde mit dest. Wasser auf einen Feststoffgehalt von 4 Gew. % verdünnt. Die zu bindenden Antikörper wurden mit 0,05 M Natriumacetat + 0,5 M NaCl + 0,05 M Tris + 0,01% Natriumazid pH 7,0 auf einen Proteingehalt von 5 mg/ml verdünnt. 1 ml des obengenannten Polymerisats wurde mit 200 µl Antikörper-Lösung gemischt. Dann wurden 0,050 ml einer 20 %igen Lösung von Tween 20 (Fa. Merck Eurolab, Darmstadt, Deutschland) hinzugegeben und das Ganze nochmals gemischt. Hierzu wurde 0,025 ml 1 N HCl zugegeben, so daß ein pH-Wert von ca. 3 erreicht wurde. Nach einer Inkubationszeit von 30 min. bei Raumtemperatur wurde 0,25 ml 1 M Phosphatpuffer pH 6,5 und 0,25 ml Natriumcyanborhydrid-Lösung (25 mg/ml) hinzugesetzt und gut durchgemischt. Anschließend erfolgte eine Inkubation von einer Stunde bei Raumtemperatur.

Dieser Beladungsansatz wurde sodann 30 min. bei ca. 50 000 g zentrifugiert. Der Überstand wurde verworfen. Der Rückstand wurde in 4 ml Imidazolpuffer pH 8,1 (5g/L Imidazol, 40g/L Saccharose, 1g/L Humanalbumin) resuspendiert. Anschließend erfolgte eine Ultraschall-Behandlung (Bronson Sonyfier B15) für 30 Sekunden. Das so redispergierte Reagenz wurde im Volumenverhältnis 1:7,5 mit dem zuvor genannten Imidazolpuffer verdünnt und nochmals 30 Sekunden mit Ultraschall behandelt.

### (ii) Herstellung eines Standards/Kontrolle

Der Proteingehalt des nach Expression und Aufreinigung erhaltenen rekombinanten pCT wurde mittels einer Proteinbestimmung nach Lowry et al. (1951, J.Biol.Chem. 193, 265-275) in der Präparation bestimmt. Zur Herstellung eines Standards wurde eine geeignete Menge dieser Präparation in phosphatgepufferter Kochsalzlösung mit 10g/l Rinderserum-Albumin aufgenommen und der Gehalt an rekombinanten pCT berechnet.

### (iii) Nachweis von pCT

Die nach Beispiel 7(i) durch Bindung des offenbarungsgemäßen monoklonalen Antikörpers gegen Calcitonin und des offenbarungsgemäßen monoklonalen Antikörpers gegen Katacalcin an Latexpartikel hergestellten Reagenzien wurden in einem Volumenverhältnis 1+1 gemischt und zur Messung von pCT in einem Standard und in Patientenseren am Behring Nephelometer Analyser (BNA, Dade Behring, Marburg, Deutschland) eingesetzt. Das gemischte Reagenz wird bei Mischung mit pCT- haltigen Proben agglutiniert. Die Intensität des Streulichts im BNA ist von der pCT-Konzentration der Probe abhängig, so dass durch Vergleich mit Verdünnungen eines Standards bekannter Konzentration die pCT-Konzentration in der Probe ermittelt werden kann. Die erforderlichen Verdünnungen des Standards mit N-Diluens (Dade Behring, Marburg, Deutschland) werden automatisch vom Behring Nephelometer Analyser erstellt. Das Messergebnis wird automatisch anhand einer Logit-log-Funktion berechnet. Zur Messung wurden 100 µl Probe oder Standard mit 100 µl N-Diluens (Dade Behring, Marburg, Deutschland) und mit 40 µl des gemischten Reagenzes in der Reaktionskuvette gemischt und nach 12 min. die Veränderung des Meßsignals (in Bit) am BNA gemessen. Die Ergebnisse sind in Tabelle 4 zusammengefasst.

**Tabelle 4 : Standardkurve und Messung von Proben**

| **Probe** | **Meßsignal BNA in Bit** | **ng pCT/ml** |
|---|---|---|
| Standardverdünnung 1 | 2727 | 250* |
| Standardverdünnung 2 | 1972 | 125* |
| Standardverdünnung 3 | 1152 | 62,5* |
| Standardverdünnung 4 | 450 | 31,3* |
| Standardverdünnung 5 | 157 | 15,6* |
| Standardverdünnung 6 | 54 | 7,8* |
| Standardverdünnung 7 | 26 | 3,9* |
| Standardverdünnung 8 | 14 | 2,0* |

| Normalserum | -188 | < 2,0 |
|---|---|---|
| pCT-haltiges Serum 1 | 219 | 19,3 |
| pCT-haltiges Serum 2 | 770 | 46,3 |
| pCT-haltiges Serum 3 | 1594 | 90,7 |
| pCT-haltiges Serum 4 | 627 | 39,6 |
| pCT-haltiges Serum 5 | 43 | 6,3 |

| | | |
|---|---|---|
| *Berechnet auf Basis des eingesetzten Standards | | |

### 8. Herstellung einer stabilen pCT-Lösung

### (i) Herstellen der pCT- Lösung

Zur Herstellung der insbesondere als Standard und/oder Kontrolle geeigneten pCT-Lösung wurde eine geeignete Menge des nach Expression und Aufreinigung erhaltenen rekombinanten pCT in verschiedenen Matrizes aufgenommen.

### Matrix 1:

Phosphatgepufferter Kochsalzlösung pH 7,2 + 1g/l NaN₃ + 40g/l Polygeline (Hoechst Marion Roussel Deutschland GmbH, Prod.Nr.: 125590) + 80000 KIE/I Antagosan® (Wirkstoff: Aprotinin, Hoechst Marion Roussel Deutschland GmbH, Prod.Nr.: 122162)

### Matrix 2:

Phosphatgepufferter Kochsalzlösung pH 7,2 + 1 g/l NaN₃ + 40g/l Polygeline (Hoechst Marion Roussel Deutschland GmbH, Prod.Nr.: 125590) + 80000 KIE/I Antagosan® (Wirkstoff: Aprotinin, Hoechst Marion Roussel Deutschland GmbH, Prod.Nr.: 122162) + 10g/l Cholesterol, water soluble (Fa. Sigma, Best.Nr: C-1145)

### Matrix 3:

Lipoproteinfreies Human-Citratplasma (Herstellung gemäß Beispiel 4 von EP-0 606 616) + 1g/l NaN₃ + 80000 KIE/I Antagosan^{®} (Wirkstoff: Aprotinin, Hoechst Marion Roussel Deutschland GmbH, Prod.Nr.: 122162)

### Matrix 4

Lipoproteinfreies Human-Citratplasma (Herstellung gemäß Beispiel 4 von EP-0 606 616) + 1g/l NaN₃ + 80000 KIE/I Antagosan^{®} (Wirkstoff: Aprotinin, Hoechst Marion Roussel Deutschland GmbH, Prod.Nr.: 122162) + 10g/l Cholesterol, water soluble (Fa. Sigma, Best.Nr: C-1145)

### (ii) Überprüfung der Stabilität

Zur Überprüfung der Stabilität der pCT-Lösung wurde diese bei +2°C bis +8°C eingelagert und nach unterschiedlich langen Lagerzeiten die Veränderung des Meßsignals (in Bit) im pCT-Nachweisverfahren gemäß Beispiel 7(iii) ermittelt. Die Ergebnisse sind in den Tabelle 5 zusammengefaßt.

**Tabelle 5: Lagerstabilität von pCT-Lösungen**

| | pCT-Lösung (125 ng/ml) | | | | Abweichung in % zu Messsignal BNA in Bit nach Herstellung |
|---|---|---|---|---|---|
| | Messsignal BNA in Bit nach Herstellung | Messsignal BNA in Bit nach 3 Wochen Lagerung bei +2°C - +8°C | Abweichung in % zu Messsignal BNA in Bit nach Herstellung | Messsignal BNA in Bit nach 8 Wochen Lagerung bei +2°C - +8°C | |
| Matrix 1 | 1629 | 489 | -70 | - | |
| Matrix 2 | 1916 | 1959 | 2,2 | 1821 | -5,0 |
| Matrix 3 | 1840 | 1321 | -28,2 | - | - |
| Matrix 4 | 1757 | 1324 | -24,6 | - | - |

Ergebnis: Die auf der Matrix 2 basierende pCT-Lösung ist besonders stabil. Der Zusatz von Cholesterol fördert auch die Stabilität von pCT in Serum/Plasma-Matrix.

## Patentansprüche

1. Lösung enthaltend (I) ein Polypeptid oder mehrere Polypeptide, worin besagte Polypeptide die Aminosäuresequenz des menschliches Procalcitonins oder die Aminosäuresequenz oder die Aminosäuresequenz enthalten und (II) einen oder mehrere Sterinester der allgemeinen Formel I:
R₁-O-(O)C-R₂-C(O)-O-[CH₂-CH₂-O]ₙ-CH₂-CH₂-OH Formel I
worin n = 1-200 und
R₁ = Sterin ist und
R2 = einen aliphatischen oder aromatischen Ring mit 4 bis 8 C-Atomen, von denen mindestens eines durch N, S oder O ersetzt werden kann, oder eine geradlinige oder verzweigte Kette mit 0 bis 12 C-Atomen darstellt

2. Lösung nach Anspruch 1, wobei R₁ eine Verbindung der allgemeinen Formel II ist: 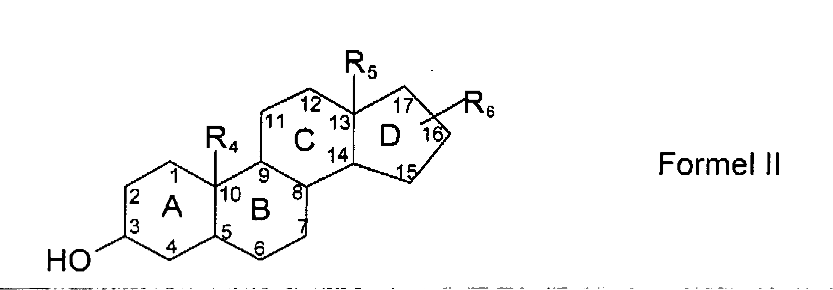 R₄ und R₅ H oder -CH₃ sind,
R₆ eine geradkettige oder verzweigte Kette mit 1 bis 12 C-Atomen, eine -OH oder =O Gruppe ist,
die Ringe A, B, C und D jeder für sich gesättigt, ungesättigt, oder aromatisch sind,
und, wenn R₄ = -C(19)H₃ ist, der Ring B zwischen C(9) und C(10) unter Ausbildung einer Doppelbindung zwischen C(9) und C(19) geöffnet ist.

3. Lösung nach Anspruch 1, wobei R₁ ausgewählt ist aus der Gruppe: Cholesterin, Vitamin D3, Stigmasterin und Oestron.

4. Lösung nach einem der Ansprüche 1-3, wobei die Konzentration des Sterinesters 0.05 Gew.% bis 5 Gew,% beträgt.

5. Lösung nach einem der Ansprüche 1 -4, wobei die Polygeline-Konzentration 0.1 Gew% bis 10 Gew% beträgt.

6. Kontrolle und/oder Standard enthaltend eine Lösung gemäß einem der vorhergehenden Ansprüche.

## Claims

1. A solution comprising (1) a polypeptide or a plurality of polypeptides, wherein said polypeptides comprise the amino acid sequence of human procalcitonin or the amino acid sequence or the amino acid sequence and (II) one or more sterol esters of the general formula I:
R₁-O- (O)C-R₂-C(O)-O- [CH₂-CH₂-O]ₙ-CH₂-CH₂-OH Formula I
where n = 1-200 and
R₁ is sterol, and
R₂ is an aliphatic or aromatic ring of 4 to 8 carbon atoms, at least one of which may be replaced by N, S or 0, or is a straight or branched chain of 0 to 12 carbon atoms
And (III) polygeline.

2. A solution as claimed in claim 1 where R₁ is a compound of the general formula II: 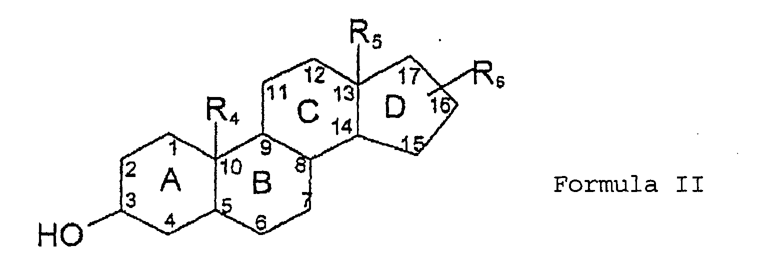 where R₄ and R₅ are H or -CH₃,
R₆ is a straight-chain or branched chain of 1 to 12 carbon atoms, an -OH or =O group,
the rings A, B, C and D are each saturated, unsaturated or aromatic,
and, if R₄ is -C(19)H₃, ring B is opened between C(9) and C (10) with formation of a double bond between C(9) and C(19).

3. A solution as claimed in claim 1 wherein R₁ is selected from the group: cholesterol, vitamin D3, stigmasterol and estrone.

4. A solution as claimed in one of claims 1-3, wherein the sterol ester concentration is from 0.05% by weight to 5% by weight.

5. A solution as claimed in one of claims 1-4, wherein the polygeline concentration is from 0.1% by weight to 10% by weight.

6. A control and/or standard comprising a solution as claimed in one of the preceding claims.

## Revendications

1. Solution contenant (I) un polypeptide ou plusieurs polypeptides, dans laquelle lesdits polypeptides contiennent la séquence d'acides aminés de la procalcitonine humaine : ou la séquence d'acides aminés : ou la séquence d'acides aminés et (II) un ou plusieurs esters de stérol de formule générale I :
R₁-O-(O)C-R₂-C(O)-O-[CH₂-CH₂-O]ₙ-CH₂-CH₂-OH Formule I
où n = 1-200, et
R₁ = stérol, et
R₂ = un cycle aliphatique ou aromatique avec 4 à 8 atomes de carbone, dont au moins un peut être remplacé par N, S ou O, ou présente une chaîne linéaire ou ramifiée avec 0 à 12 atomes de carbone
et enfin (III) de la polygéline.

2. Solution selon la revendication 1 dans laquelle R₁ est un composé de formule générale II : 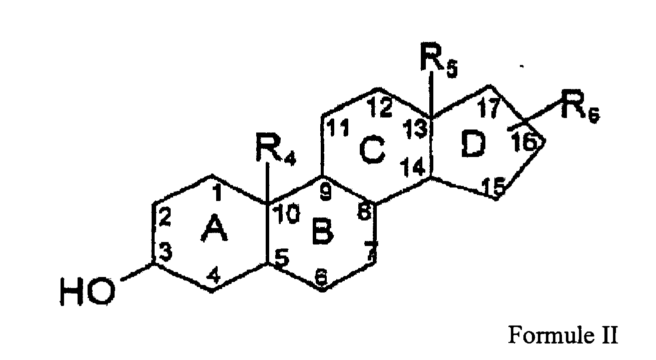 R₄ et R₅ sont H ou -CH₃,
R₆ est une chaîne linéaire ou ramifiée avec 1 à 12 atomes de carbone, un groupement -OH ou =O,
les cycles A, B, C et D sont chacun indépendamment saturés, insaturés ou aromatiques,
et si R₄ = -C(19)H₃, le cycle B est ouvert entre C(9) et C(10) en formant une double liaison entre C(9) et C(19).

3. Solution selon la revendication 1 dans laquelle R₁ est choisi dans le groupe du cholestérol, de la vitamine D₃, du stigmastérol et de l'oestrone.

4. Solution selon l'une quelconque des revendications 1 à 3 dans laquelle la concentration de l'ester de stérol est de 0,05% à 5% en poids.

5. Solution selon l'une quelconque des revendications 1 à 4 dans laquelle la concentration en polygéline est de 0,1% à 10% en poids.

6. Echantillon de contrôle ou standard contenant une solution selon l'une quelconque des revendications précédentes.
